# EUROPEAN PATENT SPECIFICATION

(11) **EP 4 537 099 B1**
(45) Date of publication and mention of the grant of the patent: **28.01.2026**
(21) Application number: 23733403.2
(22) Date of filing: 09.06.2023
(51) Int. Cl.: G01N 33/24, G01N 1/38, G01N 1/40

(54) **SOIL SAMPLE PROCESSING METHODS**
VERFAHREN ZUR VERARBEITUNG VON BODENPROBEN
PROCÉDÉS DE TRAITEMENT D'ÉCHANTILLON DE TERRE

(30) Priority: 09.06.2022 GB 202208485
(43) Date of publication of application: 16.04.2025
(73) Proprietor: Agricarbon UK Limited, Invergowrie, Dundee DD2 5EJ (GB)
(72) Inventor: ARBUCKLE, Stewart, Dundee DD25NQ (GB); GILLESPIE, Jake, Dundee DD25NQ (GB); LAW, Matthew, Dundee DD25NQ (GB); VAN DUEN, Byron, Dundee DD25NQ (GB)
(74) Representative: Murgitroyd & Company
(86) International application number: PCT/GB2023/051500
(87) International publication number: WO 2023/237891

(56) References cited:
- THAPA VESH R ET AL: "Response of soil organic matter to cover cropping in water-limited environments", GEODERMA, vol. 406, 115497, 1 October 2021 (2021-10-01), pages 1 - 12, XP086853351, ISSN: 0016-7061, [retrieved on 20211001], DOI: 10.1016/J.GEODERMA.2021.115497
- YU QIAN ET AL: "A geochemical study of toxic metal translocation in an urban brownfield wetland", ENVIRONMENTAL POLLUTION, BARKING, GB, vol. 166, 27 February 2012 (2012-02-27), pages 23 - 30, XP028479562, ISSN: 0269-7491, [retrieved on 20120309], DOI: 10.1016/J.ENVPOL.2012.02.027
- EZE SAMUEL ET AL: "Soil organic carbon stock and fractional distribution in upland grasslands", GEODERMA, ELSEVIER, AMSTERDAM, NL, vol. 314, 21 November 2017 (2017-11-21), pages 175 - 183, XP085300513, ISSN: 0016-7061, DOI: 10.1016/J.GEODERMA.2017.11.017
- ROBLES-GONZÃ LEZ IRERI V ET AL: "A review on slurry bioreactors for bioremediation of soils and sediments", MICROBIAL CELL FACTORIES, vol. 7, no. 1, 5, 29 February 2008 (2008-02-29), pages 1 - 16, XP021036439, ISSN: 1475-2859
- ASENSIO V ET AL: "Effect of soil reclamation process on soil C fractions", CHEMOSPHERE, PERGAMON PRESS, OXFORD, GB, vol. 95, 30 October 2013 (2013-10-30), pages 511 - 518, XP028775159, ISSN: 0045-6535, DOI: 10.1016/J.CHEMOSPHERE.2013.09.108
- LEUE MARTIN ET AL: "Spatially-distributed microbial enzyme activities at intact, coated macropore surfaces in Luvisol Bt-horizons", SOIL BIOLOGY AND BIOCHEMISTRY, vol. 156, 108193, 4 March 2021 (2021-03-04), pages 1 - 13, XP086539208, ISSN: 0038-0717, [retrieved on 20210304], DOI: 10.1016/J.SOILBIO.2021.108193

## Description

### FIELD

The present invention relates to methods for processing cores of soil from the earth, for subsequent analysis.

### BACKGROUND

Cores of soil, typically at depths of up to 1 metre are taken for various analytical purposes including to determine the type and quality of the soil, in particular bulk density, texture, mineral content, the nitrogen content and the organic and/or inorganic carbon content. Sampling techniques generally involve pushing or drilling a core collecting tool, including a sample tube, into the ground.

A core of soil is collected in the sample tube and subsequently removed from the tool for inspection and/or analysis. To enable useful results, it is important that a sufficient number of cores are taken across the area being surveyed and that the cores are handled carefully and in a consistent manner throughout the procedure.

Where core samples are to be analysed, a typical preliminary step is to cut the core into depth segments or samples, which are then dried to remove at least some of the moisture content. Dried samples of core material are then gently crushed ('crumbled') and sieved to remove stones or other large solids, and thereby provide a sieved sample of dried soil. The sieved sample of dried soil is representative of the soil originally in core samples that have been processed together. If desired, sieved samples of dried soil can be blended to give a representative average sample from several cores. A sieved soil sample can then be further processed as required. For example, the sieved sample may be pulverised to a small particle size and small aliquots taken for a combustion analysis, when a soil carbon content is to be determined.

Where a soil is cohesive (e.g., a clay), dried samples of core can be hard and so difficult to crumble. Typically, manual crushing e.g., in a pestle and mortar, would be employed. Whichever technique is employed, care is required to produce consistent results. The application of excessive force to core samples may break stones within the sample, resulting in the addition of small stone fragments to the sieved soil sample. This would distort the results of an analysis intended to report on the basis of only the soil content in the original core sample.

Where a large throughput of core samples is to be handled, the current techniques for the preparation of sieved samples of dried soil from core samples are laborious and may lack consistency. Large throughput of core samples also require drying facilities to allow the samples to dry completely prior to sieving. Furthermore, if a sub-sample is treated with acid, two drying stages are required (one to dry the full sample and one to dry the acidified sub-sample).

THAPA ET AL: "Response of soil organic matter to cover cropping in water-limited environments", GEODERMA, vol. 406, article number 115497, 1 October 2021, pages 1-12 discloses a method of determining the soil organic matter components of soil samples comprising the steps of dispersing a weighed portion of soil in liquid to form a slurry and passing the slurry through a 53-µm sieve.

### SUMMARY

According to a first aspect the present invention provides a method of analysing soil samples, the method comprising:
dispersing a weighed portion of soil in liquid to form a slurry;
sieving the slurry to remove particles above a selected size range;
homogenising the slurry;
taking an aliquot of the homogenised slurry for analysis; and
carrying out an analysis procedure on the aliquot of homogenised slurry to determine a characteristic of the soil.

The steps of dispersion of the portion of soil in liquid: and sieving to remove particles above a selected size range may be carried out in a generally simultaneous fashion. For example, a portion of soil is placed in a sample holder with a mesh or other perforated material forming a wall or bottom part and acting as a sieving part. A liquid (typically water) is added gradually to the portion of soil, with agitation, to separate soil particles from each other and larger particles such as stones. The soil particles pass through the mesh or other perforated material together with the liquid and are collected in a slurry collecting container. Larger particles are retained in the sample holder. The collection of soil particles that can pass through the mesh may be completed by continuing to wash through the mesh or other perforated material with liquid, for example with a pressurised jet of liquid.

The portion of soil may be from a soil core, typically a length of a soil core sample of a selected depth range. For example, of soil from 0 to 15cm, 15 to 30cm depth, and so on, down the length of the core sample. The method may include the step of collecting a portion of soil from a core sample for dispersion in liquid to form the slurry. Conveniently the soil core sample may be provided for division into portions by being presented on a tray, such as the core carrying tray as described in the applicants pending international patent application PCT/EP2021/084038, post-published as WO2022/117757. Such arrangements can allow efficient and accurate division of a core sample into portions; either manually or by an automated or semi-automated system.

Conveniently the length of a soil core or part of a soil core is noted. Together with the known diameter of the soil core, this enables bulk density measurement of the soil being analysed.

The liquid used to disperse the portion of soil to form a slurry may be water, but other liquids may be used. The liquid may be selected to be free or substantially free of the analyte or analytes that are the target of the analysis procedure. Alternatively, allowance may be made in the analytical method for a quantity of target analyte known to be present in the added liquid. The amount of liquid added may be adjusted depending on the type of soil and its moisture content. Typically, a ratio of 1:1 water to portion of soil, by weight, may be used. However, other proportions may be employed, for example more water, say from the range of 1:1 to 5:1 water to soil. In some cases, for example with notably wet soil, a lower water to soil ratio may be employed.

The step of sieving the slurry is conveniently carried out before the homogenisation procedure. For soil analysis to determine carbon content, larger particles (typically stones) above 2mm mesh size are removed. In typical analyses, the larger particle or stone content is determined by weight (or volume) to allow calculation of the 'stone content' of the original portion of soil. A volume measurement of stone content can be used for bulk density calculations of the stone content.

The step of homogenising the slurry may be carried out by grinding the solid particles of soil in the sieved slurry to obtain particles that are sufficiently fine to remain suspended in the liquid, at least until a representative aliquot can be taken. In addition or alternatively , the sieved slurry may also be homogenised by adding a suspension agent that suspends the soil particles throughout the liquid, at least until a representative aliquot can be taken. Advantageously, the use of a suspension agent avoids such a mechanically demanding grinding step during homogenisation.

A weighed quantity of suspension agent can be added to aid analysis calculations.

According to the present invention, suitable suspension agents comprise clay minerals. For example, a bentonite clay has been found effective, at least in aqueous based slurries. Bentonite may be advantageous owing to the clay's low carbon content, high abundance, low cost, and efficacy as a suspending agent. Commercially available bentonites can be used. One or more of sodium bentonite and calcium bentonite may be used, with sodium bentonite preferred.

The sieved slurry has a known weight of a suspension agent added, and a suspension formed by agitation, for example with a high shear mixer. Using a high shear mixer may also be advantageous as it further breaks down the soil aggregates and larger particles. According to the present invention, the clay is selected to be free or substantially free of the analyte or analytes that are the target of the analysis procedure.

Where a clay such as sodium bentonite is employed, it may be added to the mixture at a rate of say 5 to 15%, or even 8 to 10%, by mass of the mass of water (or other liquid) added to the portion of soil.

After homogenisation an aliquot of the homogenised slurry is taken for analysis. For analysis such as for carbon content by a combustion analyser, relatively small aliquots can be used. For example, less than 1 ml or even less than 0.5 ml. More than one aliquot may be taken to allow at least one of: repeat analyses; analyses for different analytes (that may use different techniques); or to allow retention of an unanalysed aliquot for subsequent confirmation of a result, or investigation of an anomaly in results.

The aliquot or aliquots may be subject to additional treatment before an analytical technique is applied. Drying the aliquot to produce a dried soil sample is a typical example of an additional treatment. Conveniently aliquots in suitable containers such as crucibles can be dried in a conveyor oven. Carbon content analysis is normally carried out on dried soil samples, for example by a combustion analysis, such as heating in air or oxygen to produce carbon dioxide from the carbon present in a dried sample of soil. measurement of the amount of carbon dioxide produced allows calculation of soil carbon content.

Where the organic carbon content of a soil is to be determined the aliquot or aliquots of homogenised slurry may be treated before drying with a mineral acid (typically aqueous hydrochloric acid, for example 10% by weight HCl) to convert inorganic carbon to carbon dioxide. The carbon dioxide is rapidly released from the aliquot to leave organic carbon in the dried sample for determination by the combustion analysis. Alternatively, where the total carbon content of a soil is to be determined, the aliquot or aliquots of homogenised slurry may be dried to produce a dried soil sample, without an acid treatment. A combustion analysis can then be used to allow calculation of the total carbon content of the soil.

With such methods the aliquots are typically weighed when taken, and also after drying, to allow calculation of the quantity of analyte present in the original weighed portion of soil, and for bulk density calculations.

Thus, for example, the determination of the organic carbon content of a portion of soil may involve:
taking an aliquot of the homogenised slurry;
weighing the aliquot in a container;
treating the aliquot with an acid to evolve carbon dioxide from inorganic carbon present in the aliquot;
drying the aliquot to produce a dried soil sample with inorganic carbon removed;
weighing the dried soil sample; and
carrying out an analysis, such as a combustion analysis, of the dried soil sample to thereby determine the organic carbon content of the soil.

The acid treatment causes reaction with carbonate in the aliquot to release carbon dioxide, and form salts of the acid. For example, chloride salts if hydrochloric acid treatment is used. Therefore, the dried weight of an acid treated aliquot will not be the same as that of the dried weight of an aliquot that has not been acid treated. Whilst the change in mass of a dried aliquot caused by of conversion of e.g. carbonate to chlorides can be estimated, it has been found that accuracy and consistency in results can be affected. Therefore, it is preferred that a second aliquot, that is not acid treated, is taken from the same homogenised slurry sample and dried. The dry weight of soil in the aliquot not treated with acid can be used for bulk density calculation. This bulk density value may be referred to as fine bulk density. It can be combined with the bulk density value for stone content to provide a total bulk density value. Thus, one of the characteristics determined by analysis may be bulk density, such as fine bulk density, stone bulk density, total bulk density, or combinations thereof. For example, a calculation of % dry soil per unit volume, or ratio of dry soil mass per unit volume, of the original (wet) soil sample may be undertaken. The second aliquot can be stored for subsequent examination if desired or required, for example in the event of a failure in the testing of the acid treated aliquot or to investigate unexpected or anomalous results.

Comparison of the organic carbon content obtained in this way with that of a total carbon content, obtained by processing an aliquot of the homogenised slurry without the acid treatment step discussed above, can allow determination of the inorganic carbon content of the soil.

Weighing the aliquot of homogenised slurry can be done when dispensing the aliquot into a suitable container (e.g. a crucible). Drying aliquots of the homogenised slurry can be carried out in various ways. Conveniently the aliquots in containers such as crucibles can be dried in a conveyor oven. The oven temperature and transit time through the heating zone can be adjusted to suit the analysis required. For samples in an aqueous homogenised slurry an oven temperature of about 90 to 135 degrees C with transit time of from 40 to 80 minutes may be employed. For example, 115 degrees C for 60 minutes.

The containers of aliquots are carried by a conveyor, such as a conveyor belt, through an oven. The oven may include forced extraction to remove vapourised liquid, and any gases evolved from the aliquots during drying. Such gases may include carbon dioxide and acid vapours (e.g. HCl). As acid fumes are present during drying it has been found advantageous to use conveyor oven arrangements where oven parts are made of stainless steel with acid resistant coating and/or the oven does not use recirculated air, but has fresh hot air passing through, to continually remove acid fumes.

On exiting the oven, the containers of dried aliquots i.e. dried soil samples, can be weighed and their contents then analysed, e.g. in a combustion analyser for carbon content. The combustion analyser can be a commercially available machine. These operate at a temperature in excess of 900 degrees C, for example 1350 degrees C to combust all the carbon in the dried soil sample and allow carbon content calculations based on the carbon dioxide released.

Procedures such as described herein can allow an as obtained soil sample to be processed with only one drying step before analysis. Only small aliquots of homogenised slurry are dried. Thus, the methods described herein readily lend themselves to large numbers of samples being taken. The methods described herein allow for easier automation and improved portability of equipment (owing to the smaller machines which are required). For example, less work is involved in comparison with previous methods for organic carbon analysis, where portions of a core sample of soil are dried, crumbled, sieved to remove larger particles such as stones, and then treated with acid before drying again for combustion analysis.

For some analysis procedures the aliquot or aliquots of homogenised slurry may not be dried. For example, determination of a target analyte may be carried out by spectroscopy of the aliquot, which may then be dried for a subsequent analysis for a different target analyte.

The procedures described herein are amenable to automated processing or at least semi-automated processing. This can allow large numbers of analyses to be carried out in a relatively short timescale. For example, process steps such as weighing, adding liquids and solids, dispersing to form a slurry, obtaining an aliquot of homogenised slurry, drying and loading into an analyser machine (such as a combustion analyser) - may be carried out with the use of automatic weighing and dispensing machinery; and by making use of robotic arms or the like when transferring portions, slurries and aliquots from one process step to the next.

As discussed above, the soil core samples may be provided for division into portions by being presented on a tray. Such arrangements can allow efficient and accurate division of a core sample into portions; either manually or by an automated or semi-automated system.

In a convenient arrangement, described in more detail hereafter and with reference to an example, a camera and computer system arrangement may be used to determine an end of a soil core (e.g., the end that was at the surface of the ground being sampled). The system marks the core with a line or lines of (e.g., laser) light where a dividing cut or cuts are to be made. For example, a line 10 cm from the surface end of the core sample to allow cutting off the 0 to 10 cm depth of the core for analysis. A process operator can use the lines of light to rapidly divide soil cores into sample portions. As a further alternative, such an arrangement may be fully automated by using a robotic arm to make the cuts and remove portions of soil from cores for onwards processing.

In the methods described herein, weighed soil portions are formed into a slurry with a liquid and sieved to remove particles, such as stones, that are above a selected size range. Convenient apparatus for carrying out this part of the method constitutes another aspect of the invention.

An apparatus for preparing a slurry of a soil sample not forming part of the claimed invention is provided. The apparatus comprises:
a sample holder for a portion of soil, including a sieving part;
a source of liquid for supplying liquid into the sample holder;
a slurry container for collecting a slurry of soil and liquid passing through the sieving part; and
an agitator means for agitating a said portion of soil and liquid from the source in the sample holder.

The apparatus comprises a sample holder for a portion of soil which may have a mesh or other perforated material forming a wall or bottom part, acting as the sieving part. The mesh or other perforated material (e.g., perforated sheet material such as perforated steel) may form at least part of the bottom of the sample holder; and may be releasably attached to allow cleaning or replacement. The sample holder may be nestable inside the slurry collection container. This aids collection of all of the liquid and soil particle slurry that passes through the sieving part. The sample holder can be removable or partially removable from the slurry collection container during processing to assist "washing through" remaining soil particles from the sample holder into the collection container, using additional liquid.

The apparatus includes an agitator means for agitating the sample and liquid to form a slurry. Thus, the apparatus may include an agitator brush for insertion into the sample holder to agitate the soil sample and added liquid to form the slurry. The agitator brush may be motor driven, for example to rotate and/or reciprocate up and down within the sample holder. Alternative agitation means are contemplated, for example an ultrasound transducer may be employed to separate and disperse soil particles in the liquid.

The apparatus further includes a source of liquid for adding to the sample holder to make the slurry. Typically, the liquid is water and may be supplied at least as a relatively gently flow into the sample holder while agitation is proceeding. For example, the liquid such as water may be supplied via a hose. A jet or spray arrangement may be provided to provide a more forceful stream of liquid. The jet or spray may be employed to complete washing of soil particles through the sieving part and/or to clean down the inside of the sample holder and/or agitator arrangement.

The liquid added to the sample of soil may be measured to determine the weight added as it is introduced into the sample holder, or it may be calculated by obtaining the weight of the slurry formed in the slurry collection container and the weight of particles retained in the sample holder.

The apparatus may be used as follows. A portion of soil is introduced to the sample holder (either after weighing or to be weighed in the sample holder). The sample holder is nested within the slurry container. A stream of liquid such as water is introduced into the sample holder and an agitator brush inserted and rotated to break up the sample of soil into a slurry. The slurry of soil and liquid passes through the sieving part, with larger particles retained by the sieving part. The brush is removed from the sample holder, optionally whilst liquid is used to wash adhering soil into the sample holder.

The sample holder may then be partially or completely removed from the slurry collection container and a wash down procedure carried out by spraying liquid at high pressure into the sample holder to wash remaining particles of soil through the sieving part into the slurry collection container, whilst also cleaning any remaining stones. This method may be achieved using a pressure washer with a rotating pencil jet nozzle.

Thereafter the slurry collected in the slurry collection container can be weighed, and homogenised e.g., by treating with a suspension agent and mixing, typically with a high shear mixing agitator. Conveniently such process steps are carried out in the slurry collection container to ensure that the homogenised slurry is fully representative of the original sample of soil, after sieving to remove larger particles.

The apparatus for preparing a slurry of a soil sample, can be used in an automated or semi-automated way.
To aid throughput, the apparatus may be formed to allow processing of more than one portion of soil at a time.

Another apparatus for preparing a slurry of a soil sample not forming part of the claimed invention is provided, said apparatus comprising:
a) a sample loading and wash down station comprising a supply of liquid;
b) at least two processing stations for slurry preparation, each comprising:
   a sample holder for a portion of soil, including a sieving part;
   a source of liquid for supplying liquid into the sample holder;
   a slurry container for collecting a slurry of soil and the liquid passing through the sieving part; and
   an agitator means for agitating a sample of soil and supplied liquid in the sample holder;

wherein each sample holder is moveable into and out of nesting engagement within a respective one of the slurry containers and
wherein each sample holder and respective slurry container is moveable to the sample loading and wash down station.

The operation of such an apparatus is discussed further hereafter by way of example only, and with reference to a particular embodiment.

### BRIEF DESCRIPTION OF THE DRAWINGS

Figure 1 shows a flow chart for an exemplary process to prepare and analyse soil samples for carbon content;
Figure 2 shows, in schematic perspective view, apparatus for aiding sample preparation;
Figures 3A, 3B and 3C show, in schematic plan views, elements of apparatus for preparing slurries of soil; and
Figure 4 shows an apparatus for preparing slurries of soil.

### DETAILED DESCRIPTION OF THE DRAWINGS

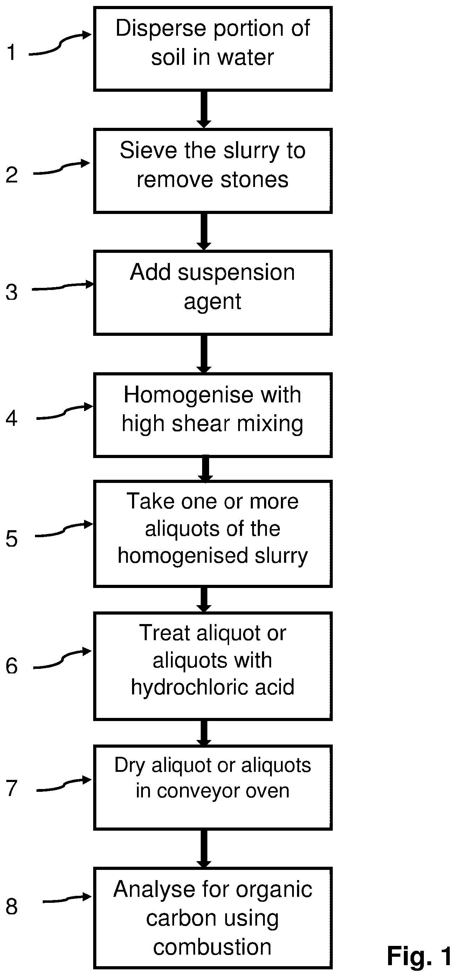

A flow chart for an exemplary method of determining the organic carbon content of a soil is shown in figure 1. Firstly, (step 1) a weighed portion of soil from a core sample is dispersed in water, typically at a 1:1 ratio by weight. The slurry of soil water is then sieved to remove stones or other particles of >2mm at step 2. The stone content can be determined by weighing the particles of >2mm.

A bentonite suspension agent is added at 3, (for example at 8% by weight of the added water) and the mixture homogenised using a high shear mixer at step 4. An advantage of using a suspension agent such as bentonite is that it may avoid a mechanically demanding grinding step which would normally be part of the homogenisation.

In some examples, additionally to adding a suspending agent (e.g. bentonite), it may be suitable to grind the wet slurry after sieving as smaller particles will sit in suspension better so less or no need for suspending agent.

The homogenised slurry can then be sampled (step 5), with each aliquot representative of the original portion of soil. Typically, at least two aliquots are taken. One or more of the aliquots can be preserved in a capped or sealed container, for use in the event that a repeat analysis is required, for example if an anomalous result is obtained, or the rest of the process fails for some reason. Additionally, or alternatively an aliquot may be dried, without the acid treatment described below. The dry weight of this non-acid treated aliquot may be used in the determination of the bulk density of the original soil.

The aliquot or aliquots to be analysed are then treated, in step 6, with hydrochloric acid to convert inorganic carbonate such as calcium carbonate, to carbon dioxide.

Drying in a conveyor oven (step 7) with extraction to remove acid and carbon dioxide, provides one or more dried soil samples with the inorganic carbon removed.

In step 8 the sample or samples are analysed in a combustion analyser to allow determination of the organic content of the soil.

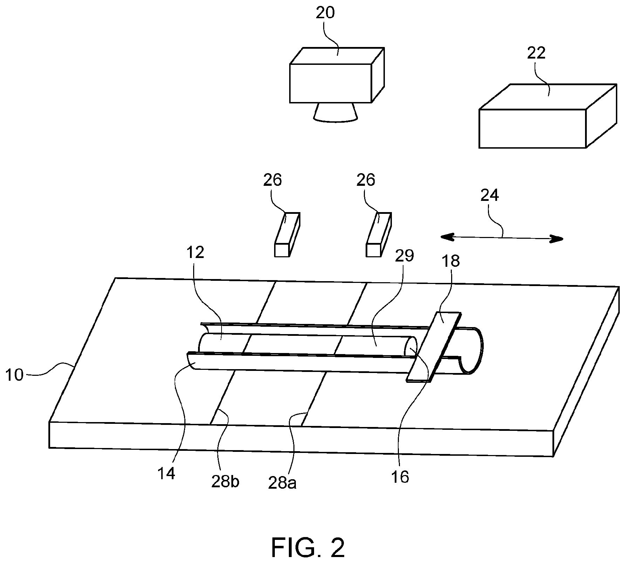

Figure 2 shows in schematic perspective a method of dividing soil core into portions of soil core for analysis.

Table 10 supports a soil core 12 in a part cylinder tray 14. The soil core 12 has an end 16 that was at the surface of the ground being sampled. A datum marker 18, in the form of a coloured piece of card has been placed at the end 16.

A camera 20 and associated computer controller 22 detects the datum marker 18. As suggested by double headed arrow 24 the controller 22 directs automatic relative motion between lasers 26 and datum marker 18. For example, the lasers 26 are moved along tracks (not shown); or the table 10 moves relative to the lasers. Lasers 26 project lines of light 28A, 28B across the table 10 and core 12. These lines mark core 12 into sections, e.g. the surface to 15cm depth section (i.e. portion of soil 29). A cut made in the core 12 along the line 28A allows portion 29 to be removed for analysis.

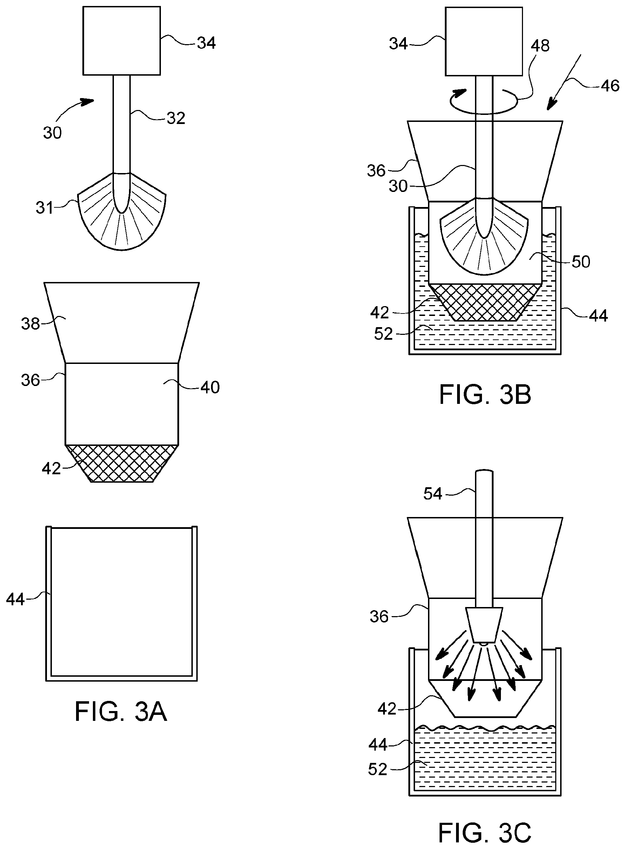

Figure 3A shows in schematic plan view elements of an apparatus for preparing a slurry of a soil sample. An agitator brush 30 has a brush head 31 mounted for rotation by shaft 32 connecting to motor 34. Sample holder for a portion of soil 36 has a conical top section 38 a cylindrical section 40 and a sieving part 42. Sieving part 42 is a detachable conical perforated steel part, that will allow passage of particles of < 2mm diameter. The sieving part may be detachable by means of a screw connection to the rest of the sample holder 36. Slurry container 44 is a cylindrical container that is sized to receive at least cylindrical section 40 and a sieving part 42 of sample holder 36.

Figures 3B and 3C show the use of the parts depicted in figure 3A. As shown in figure 3B, after insertion of a sample of soil into sample holder 36 is nested within slurry container 44, and brush 30 is inserted into the sample holder, together with a flow of water suggested by arrow 46. Rotation (suggested by arrow 48) of the brush 30 results in a soil/water slurry 50 being formed in the sample holder 36. Slurry passing out of the sample holder 36 through sieving part 42 collects as sieved slurry 52 in the slurry container 44.

Figure 3C shows the apparatus of figure 3B, but with brush 30 removed and sample holder 36 partially removed from slurry container 44. A spray cleaner lance 54 is being used to wash down the inside of sample container 36, with water and remaining particles of soil that pass through the sieving part 42 (shown without an indication of the perforations in this view) continuing to be collected as part of sieved slurry 52.

The sieved slurry 52 shown collected in figure 3C can then follow through the rest of the process as depicted in figure 1, starting with homogenisation by addition of a suspension agent.

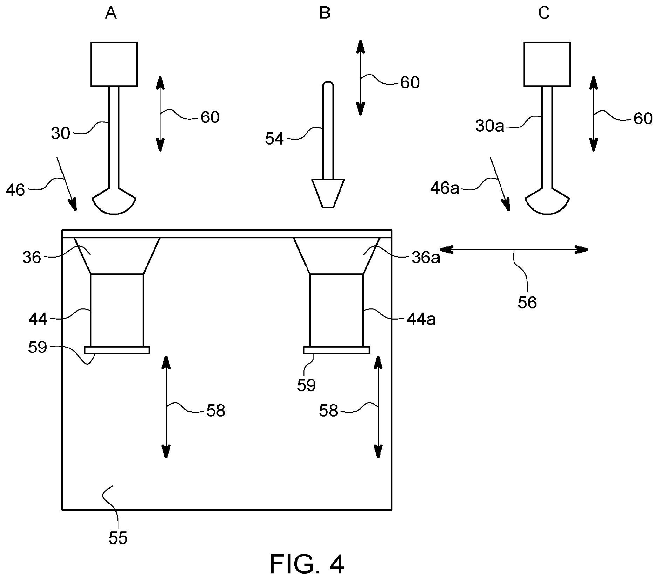

Figure 4 shows schematically an apparatus for preparing a slurry of a soil sample, generally in the manner described above, but with improved throughput provided by the use of two each of brushes 30,30a; sample containers 36,36a;slurry containers 44,44a; and water supplies 46,46a. There are three processing stations A, B and C as shown in the drawing. Stations A and C are for slurry preparation, and station B for sample loading and wash down procedures, as discussed below.

Sample containers 36, 36a are shown nested in slurry containers 44, 44a respectively to form container assemblies 36,44 and 36a,44a. Both sample containers 36, 36a and slurry containers 44, 44a are mounted to frame 55 for transverse motion as suggested by double headed arrow 56.

This allows either of sample holder and slurry container assemblies 36,44 or 36a,44a to be moved to station 'B' shown in the figure, where spray cleaner lance 54 is located. As suggested by double headed arrows 58 the slurry containers 44,44a are supported on platforms 59 that can be raised or lowered to move slurry containers 44,44a into or out of nesting engagement with the corresponding sample container 36,36a. Brushes 30,30a and spray lance 54 can likewise be raised or lowered (arrows 60) into or out of a sample holder placed beneath them.

The depicted arrangement can be used as follows. Starting from the position shown in figure 4 a sample of soil in sample holder 36 can be slurried by use of the brush 30 and water supply 46 in the manner depicted in figure 3B. This occurs at station A in figure 4. (That soil sample was previously loaded into holder 36 when the holder and slurry container 44 were at station B.) At the same time as the slurrying procedure is occurring at station A, a slurried sample in container assembly 36a,44a is being washed down at position B by use of the lance 54, in the manner depicted in figure 3C.

The wash down procedure at station B is faster than the procedure at station A. Therefore, while the slurry procedure at station A is in progress, several additional operations can be carried out at station B. The slurry container 44a can be removed for onwards processing of the slurry through to analysis; stones can be removed from sample container 36a; and a fresh sample of soil placed in sample holder 36a, with a replacement slurry container 44a fitted. Thus, station B is used for a slurry wash down procedure and stone removal from the sample container; followed by a replenishment with a fresh portion of soil.

Once the fresh portion of soil is in assembly 36a,44a the brush 30 is withdrawn from assembly 36,44 and the frame 55 traverses so that assembly 36a,44a is at station C and assembly 36,44 is at station B. In that position a slurry procedure using brush 30a and water supply 46a is carried out on the soil sample in assembly 36a,44a at station C.

At the same time the slurry in assembly 36,44 prepared at station A is now at station B and can be washed down using water lance 54. The slurry container 44 can be removed for onwards processing of the slurry through to analysis; stones can be removed from sample container 36; and a fresh sample of soil placed in sample holder 36, with a replacement slurry container 44 fitted. The procedure then repeats.

Thus, the apparatus of figure 4 can be used to process portions of soil in rapid succession.

## Claims

1. A method of analysing soil samples, the method comprising:
dispersing (1) a weighed portion of soil in liquid to form a slurry (50);
sieving (2) the slurry (50) to remove particles above a selected size range; homogenising (4) the slurry (50);
wherein the slurry (50) is homogenised by the addition (3) of a suspension agent and wherein the suspension agent comprises a clay;
taking (5) an aliquot of the homogenised slurry (50) for analysis;
carrying out an analysis procedure (7) on the aliquot of homogenised slurry (50) to
determine a characteristic of the soil, wherein the characteristic of the soil that is determined by analysis is a carbon content.

2. The method of claim 1 wherein:
the weighed portion of soil is placed in a sample holder (36) with a mesh or other perforated material forming a wall or bottom part (42) for sieving the slurry (50);
the liquid is added to the weighed portion of soil, with agitation; and
soil particles passing through the mesh or other perforated material together with the liquid are collected in a slurry collecting container (44).

3. The method of claim 2 wherein the collection of soil particles that can pass through the mesh is completed by washing them through the mesh or other perforated material with additional liquid, for example with a pressurised jet of liquid.

4. The method of any preceding claim further comprising drying the aliquot of the homogenised slurry (50) before carrying out the analysis procedure.

5. The method of any preceding claim wherein the portion of soil is from a soil core sample (12).

6. The method of any preceding claim wherein the liquid for dispersing the soil to form a slurry (50) is water.

7. The method of claim 6 wherein the ratio of water to soil by weight is from 1:1 to 5:1 water to soil.

8. The method of any preceding claim wherein: the slurry is sieved to remove particles above a 2 mm mesh size; or the method includes weighing the particles above the selected size range.

9. The method of any preceding claim including determining the volume of particles above the selected size range, wherein the method optionally includes calculating the density of particles above the selected size range.

10. The method of any preceding claim comprising drying the aliquot and determining dry weight of the soil in the aliquot, and optionally calculating bulk density as the ratio of dry soil mass per unit volume of the original wet soil sample.

11. The method of claim 1 wherein the suspension agent comprises a bentonite.

12. The method of any one of claims 1 to 8 wherein the solid particles of soil in the sieved slurry (52) are ground to obtain particles that are sufficiently fine to remain suspended in the liquid, at least until a representative aliquot can be taken.

13. The method of any preceding claim wherein the characteristic of the soil that is determined by analysis is the organic carbon content; and the method further comprises:
weighing the aliquot of the homogenised slurry (50) in a container (44);
treating the aliquot with an acid to evolve carbon dioxide from inorganic carbon present in the aliquot;
drying the aliquot to produce a dried soil sample with inorganic carbon removed;
weighing the dried soil sample; and carrying out an analysis of the dried soil sample to thereby determine the organic carbon content of the soil.

14. The method of claim 13 further comprising determining the inorganic carbon content of the soil by:
a) determining a total carbon content of the soil by:
weighing a second aliquot of the homogenised slurry (50) in a container
drying the second aliquot to produce a second dried soil sample;
weighing the second dried soil sample; and
carrying out an analysis of the second dried soil sample to thereby determine the total carbon content of the soil; and.
b) subtracting the organic carbon content of the soil from the total carbon content to obtain the inorganic carbon content of the soil.

15. The method of any preceding claim wherein the carbon content determination or determinations is by means of a combustion analysis.

## Patentansprüche

1. Ein Verfahren zum Analysieren von Bodenproben, wobei das Verfahren Folgendes beinhaltet:
Dispergieren (1) eines gewogenen Teils von Boden in Flüssigkeit, um eine Aufschlämmung (50) zu bilden;
Sieben (2) der Aufschlämmung (50), um Partikel über einem ausgewählten Größenbereich zu entfernen;
Homogenisieren (4) der Aufschlämmung (50);
wobei die Aufschlämmung (50) durch die Zugabe (3) eines Suspensionsmittels homogenisiert wird und wobei das Suspensionsmittel einen Ton beinhaltet;
Nehmen (5) eines Aliquots der homogenisierten Aufschlämmung (50) zur Analyse;
Durchführen eines Analyseverfahrens (7) an dem Aliquot der homogenisierten Aufschlämmung (50), um ein Charakteristikum des Bodens zu bestimmen, wobei das Charakteristikums des Bodens, das durch Analyse bestimmt wird, ein Kohlenstoffgehalt ist.

2. Verfahren gemäß Anspruch 1, wobei:
der gewogene Teil von Boden in einen Probenhalter (36) mit einem Gitter oder einem anderen perforierten Material, das eine Wand oder einen Bodenteil (42) zum Sieben der Aufschlämmung (50) bildet, platziert wird;
die Flüssigkeit dem gewogenen Teil von Boden unter Rühren zugegeben wird; und
Bodenpartikel, die zusammen mit der Flüssigkeit durch das Gitter oder das andere perforierte Material hindurchgehen, in einem Aufschlämmungssammelbehälter (44) gesammelt werden.

3. Verfahren gemäß Anspruch 2, wobei die Sammlung von Bodenpartikeln, die durch das Gitter hindurchgehen können, durch Waschen derselben durch das Gitter oder das andere perforierte Material mit zusätzlicher Flüssigkeit, zum Beispiel mit einem unter Druck stehenden Flüssigkeitsstrahl, vervollständigt wird.

4. Verfahren gemäß einem der vorhergehenden Ansprüche, das ferner das Trocknen des Aliquots der homogenisierten Aufschlämmung (50) vor dem Durchführen des Analyseverfahrens beinhaltet.

5. Verfahren gemäß einem der vorhergehenden Ansprüche, wobei der Teil von Boden von einer Bodenkernprobe (12) stammt.

6. Verfahren gemäß einem der vorhergehenden Ansprüche, wobei die Flüssigkeit zum Dispergieren des Bodens, um eine Aufschlämmung (50) zu bilden, Wasser ist.

7. Verfahren gemäß Anspruch 6, wobei das Gewichtsverhältnis von Wasser zu Boden von 1:1 bis 5:1 Wasser zu Boden beträgt.

8. Verfahren gemäß einem der vorhergehenden Ansprüche, wobei: die Aufschlämmung gesiebt wird, um Partikel über einer Maschengröße von 2 mm zu entfernen; oder das Verfahren das Wiegen der Partikel über dem ausgewählten Größenbereich umfasst.

9. Verfahren gemäß einem der vorhergehenden Ansprüche, das das Bestimmen des Volumens von Partikeln über dem ausgewählten Größenbereich umfasst, wobei das Verfahren optional das Berechnen der Dichte von Partikeln über dem ausgewählten Größenbereich umfasst.

10. Verfahren gemäß einem der vorhergehenden Ansprüche, das das Trocknen des Aliquots und das Bestimmen des Trockengewichts des Bodens in dem Aliquot und optional das Berechnen der Schüttdichte als das Verhältnis der Trockenbodenmasse pro Volumeneinheit der ursprünglichen nassen Bodenprobe beinhaltet.

11. Verfahren gemäß Anspruch 1, wobei das Suspensionsmittel einen Bentonit beinhaltet.

12. Verfahren gemäß einem der Ansprüche 1 bis 8, wobei die festen Partikel von Boden in der gesiebten Aufschlämmung (52) gemahlen werden, um Partikel zu erhalten, die ausreichend fein sind, um in der Flüssigkeit suspendiert zu bleiben, zumindest bis ein repräsentatives Aliquot genommen werden kann.

13. Verfahren gemäß einem der vorhergehenden Ansprüche, wobei das Charakteristikum des Bodens, das durch Analyse bestimmt wird, der Gehalt an organischem Kohlenstoff ist; und das Verfahren ferner Folgendes beinhaltet:
Wiegen des Aliquots der homogenisierten Aufschlämmung (50) in einem Behälter (44);
Behandeln des Aliquots mit einer Säure, um Kohlendioxid aus anorganischem Kohlenstoff, der in dem Aliquot vorhanden ist, zu entwickeln;
Trocknen des Aliquots, um eine getrocknete Bodenprobe mit entferntem anorganischem Kohlenstoff zu erzeugen;
Wiegen der getrockneten Bodenprobe; und
Durchführen einer Analyse der getrockneten Bodenprobe, um dadurch den Gehalt an organischem Kohlenstoff des Bodens zu bestimmen.

14. Verfahren gemäß Anspruch 13, das ferner das Bestimmen des Gehalts an anorganischem Kohlenstoff des Bodens durch Folgendes beinhaltet:
a) Bestimmen eines Gesamtkohlenstoffgehalts des Bodens durch Folgendes:
Wiegen eines zweiten Aliquots der homogenisierten Aufschlämmung (50) in einem Behälter,
Trocknen des zweiten Aliquots, um eine zweite getrocknete Bodenprobe zu erzeugen;
Wiegen der zweiten getrockneten Bodenprobe; und
Durchführen einer Analyse der zweiten getrockneten Bodenprobe, um dadurch den Gesamtkohlenstoffgehalt des Bodens zu bestimmen; und
b) Subtrahieren des Gehalts an organischem Kohlenstoff des Bodens von dem Gesamtkohlenstoffgehalt, um den Gehalt an anorganischem Kohlenstoff des Bodens zu erhalten.

15. Verfahren gemäß einem der vorhergehenden Ansprüche, wobei die Kohlenstoffgehaltsbestimmung oder -bestimmungen mittels einer Verbrennungsanalyse erfolgt/erfolgen.

## Revendications

1. Un procédé d'analyse d'échantillons de terre, le procédé comprenant :
disperser (1) une portion de terre pesée dans un liquide afin de former une suspension (50) ;
tamiser (2) la suspension (50) afin d'éliminer des particules dépassant une gamme de tailles sélectionnée ;
homogénéiser (4) la suspension (50) ;
dans lequel la suspension (50) est homogénéisée par l'ajout (3) d'un agent de suspension et dans lequel l'agent de suspension comprend une argile ;
prélever (5) une aliquote de la suspension homogénéisée (50) pour analyse ;
réaliser une procédure d'analyse (7) sur l'aliquote de la suspension homogénéisée (50) afin de déterminer une caractéristique de la terre, dans lequel la caractéristique de la terre qui est déterminée par analyse est une teneur en carbone.

2. Le procédé de la revendication 1 dans lequel :
la portion de terre pesée est placée dans un porte-échantillon (36) avec un maillage ou autre matériau perforé formant une paroi ou une partie inférieure (42) pour tamiser la suspension (50) ;
le liquide est ajouté à la portion de terre pesée, sous agitation ; et
des particules de terre passant à travers le maillage ou autre matériau perforé ainsi que le liquide sont recueillis dans un récipient de recueil de suspension (44).

3. Le procédé de la revendication 2 dans lequel le recueil de particules de terre qui peuvent passer à travers le maillage est complété en les arrosant pour les faire passer à travers le maillage ou autre matériau perforé avec un liquide supplémentaire, par exemple avec un jet de liquide sous pression.

4. Le procédé de n'importe quelle revendication précédente comprenant en outre faire sécher l'aliquote de la suspension homogénéisée (50) avant de réaliser la procédure d'analyse.

5. Le procédé de n'importe quelle revendication précédente dans lequel la portion de terre provient d'un échantillon de carotte de terre (12).

6. Le procédé de n'importe quelle revendication précédente dans lequel le liquide pour disperser la terre afin de former une suspension (50) est de l'eau.

7. Le procédé de la revendication 6 dans lequel le rapport eau/terre en poids va de 1/1 à 5/1 eau/terre.

8. Le procédé de n'importe quelle revendication précédente dans lequel : la suspension est tamisée afin d'éliminer des particules dépassant une taille de maille de 2 mm ; ou le procédé inclut peser les particules dépassant la gamme de tailles sélectionnée.

9. Le procédé de n'importe quelle revendication précédente incluant déterminer le volume de particules dépassant la gamme de tailles sélectionnée, le procédé incluant facultativement calculer la densité de particules dépassant la gamme de tailles sélectionnée.

10. Le procédé de n'importe quelle revendication précédente comprenant faire sécher l'aliquote et déterminer le poids sec de la terre dans l'aliquote, et facultativement calculer la masse volumique apparente en tant que rapport de la masse de terre sèche par unité de volume de l'échantillon de terre humide d'origine.

11. Le procédé de la revendication 1 dans lequel l'agent de suspension comprend une bentonite.

12. Le procédé de l'une quelconque des revendications 1 à 8 dans lequel les particules solides de terre dans la suspension tamisée (52) sont broyées afin d'obtenir des particules qui sont suffisamment fines pour rester en suspension dans le liquide, au moins jusqu'à ce qu'une aliquote représentative puisse être prélevée.

13. Le procédé de n'importe quelle revendication précédente dans lequel la caractéristique de la terre qui est déterminée par analyse est la teneur en carbone organique ; et le procédé comprend en outre :
peser l'aliquote de la suspension homogénéisée (50) dans un récipient (44) ;
traiter l'aliquote avec un acide afin de dégager le dioxyde de carbone du carbone inorganique présent dans l'aliquote ;
faire sécher l'aliquote afin de produire un échantillon de terre séché dont du carbone inorganique a été éliminé ;
peser l'échantillon de terre séché ; et
réaliser une analyse de l'échantillon de terre séché afin de déterminer de ce fait la teneur en carbone organique de la terre.

14. Le procédé de la revendication 13 comprenant en outre déterminer la teneur en carbone inorganique de la terre comme suit :
a) déterminer une teneur en carbone total de la terre comme suit :
peser une deuxième aliquote de la suspension homogénéisée (50) dans un récipient ;
faire sécher la deuxième aliquote afin de produire un deuxième échantillon de terre séché ;
peser le deuxième échantillon de terre séché ; et
réaliser une analyse du deuxième échantillon de terre séché afin de déterminer de ce fait la teneur en carbone total de la terre ; et
b) soustraire la teneur en carbone organique de la terre de la teneur en carbone total afin d'obtenir la teneur en carbone inorganique de la terre.

15. Le procédé de n'importe quelle revendication précédente dans lequel la détermination ou les déterminations de teneur en carbone se font au moyen d'une analyse par combustion.
